Europäisches Patentamt

European Patent Office

Office européen des brevets

(19)

(11) **EP 0 910 280 B1**

(12) **EUROPEAN PATENT SPECIFICATION**

(45) Date of publication and mention
of the grant of the patent:
**31.03.2004 Bulletin 2004/14**

(21) Application number: **97909546.0**

(22) Date of filing: **10.11.1997**

(51) Int Cl.⁷: **A61B 6/03**

(86) International application number:
**PCT/IB1997/001419**

(87) International publication number:
**WO 1998/023208 (04.06.1998 Gazette 1998/22)**

(54) **COMPUTER TOMOGRAPHY DEVICE FOR VOLUME SCANNING**

COMPUTERGESTEUERTER TOMOGRAPH ZUR VOLUMENABTASTUNG

DISPOSITIF DE TOMODENSITOMETRIE POUR L'EXPLORATION DE VOLUMES

(84) Designated Contracting States:
**DE FR GB NL**

(30) Priority: **27.11.1996 US 759368**
**15.10.1997 EP 97203222**

(43) Date of publication of application:
**28.04.1999 Bulletin 1999/17**

(73) Proprietor: **Koninklijke Philips Electronics N.V.**
**5621 BA Eindhoven (NL)**

(72) Inventor: **TIMMER, Jan**
**NL-5656 AA Eindhoven (NL)**

(74) Representative:
**van der Veer, Johannis Leendert et al**
**Philips**
**Intellectual Property & Standards**
**P.O. Box 220**
**5600 AE Eindhoven (NL)**

(56) References cited:
**US-A- 4 403 289**          **US-A- 5 164 973**

## Description

The invention relates to a computer tomography device which includes

- an X-ray source for irradiating an object from a plurality of directions, by means of X-rays,
- a detector system for measuring density profiles of the object from a plurality of directions,
- a reconstruction unit for deriving an image signal from the density profiles,
- the X-ray source, the detector system and the object being displaceable relative to one another in a longitudinal direction, transversely of the X-rays, during the measurement of the density profiles.

The invention also relates to a computer tomography method in which

- an X-ray source irradiates an object by means of X-rays from a plurality of directions,
- density profiles of the object are measured from a plurality of directions,
- an image signal is derived from the density profiles, and
- the object and the X-ray source are displaced relative to one another transversely of the direction of the X-rays, during the measurement of the density profiles.

A computer tomography device and a method of this kind are known from the article Computed tomography scanning with simultaneous patient translation by Carl R. Crawford and Kevin F. King in Medical Physics 17 (1990) 967-982.

Computer tomography produces images of cross-sections of the object to be examined, for example a patient to be radiologically examined. To this end, the patient is irradiated by X-rays from different directions and local differences in the X-ray absorption within the patient form density profiles for the various directions, said profiles being measured by the detector system. To this end, the X-ray source and the detector system are rotated around the patient. An image which reproduces the density of the patient in a cross-section is derived from the various density profiles. It is usually necessary to form a plurality of images of cross-sections along different, usually parallel planes. To this end, in the known computer tomography device the patient is displaced in the longitudinal direction, relative to the X-ray source and the detector system, while being irradiated by X-rays from different directions. Such a procedure is also referred to as volume scanning. Because density profiles are acquired during the uniform displacement, it is not very time consuming to measure density profiles so as to form images of a plurality of parallel cross-sections; consequently, the patient is not significantly burdened thereby. Preferably, the displacement of the patient is uniform so as to ensure that the patient does not get "travel-sick" during the measurement of the density profiles.

The measured density profiles have not been measured in a fixed longitudinal position but, because the patient is displaced while the direction wherefrom the density profiles are measured changes, the longitudinal position varies as a function of the direction within the density profiles. The density profiles are acquired along an approximately helical path, the pitch of the helix being constant when the patient is displaced at a uniform speed in the longitudinal direction. The axis of the helical path constitutes the longitudinal direction. The known computer tomography device includes a data processing unit for deriving computed density profiles from the measured density profiles, the computed density profiles always relating to a plane through the patient in a given longitudinal position. The reconstruction unit utilizes the computed density profiles so as to derive an image of the cross-section therefrom in a given longitudinal position. The computed density profiles are obtained by interpolation or extrapolation of the measured density profiles. For such interpolation the fact must be taken into account that the computed density profiles are derived from density profiles which have been measured in longitudinal positions which do not deviate excessively from the longitudinal position of the cross-section for which the image is formed. Thus, it is achieved that hardly any strip-like disturbances, also called "streaks", appear in the image.

The known computer tomography device includes a so-called symmetrical detector system. This means that the respective angles covered by the detector system to both sides of a line through the focus of the X-ray source and the center of rotation are (substantially) the same during rotation of the X-ray source and the detector system. The focus of the X-ray beam is the common point wherefrom all X-rays of the fan-shaped X-ray beam emanate (apparently). It is a drawback of such a symmetrical detector system that it involves a rather unfavorable ratio of the number of individual X-ray-sensitive detector elements to the size of the field of view of the detector system. The field of view is determined by the size of the segment covered by the detector system. It is customary to state the field of view as an angle between connecting lines from the extremities of the segment to the focus of the X-ray beam.

It is an object of the invention to provide a computer tomography device including a detector system which has a comparatively large field of view and enables execution of a volume scan yielding an image which is substantially free from disturbances. It is specifically an object of the invention to provide such a computer tomography device in which notably the appearance of streaks in the image is precluded.

This object is achieved by means of a computer device according to the invention which is characterized in that

- the detector system includes an asymmetrical system of detectors, and
- the directions wherefrom the density profiles are measured have a range of at least $4\pi$ radians.

An asymmetrical detector system extends across a segment between different angles $(-\gamma_m+\alpha,\gamma_m)$ to both sides of a line through the focus of the X-ray source and the center of rotation during the rotation of the X-ray source and the detector system. Such an asymmetrical detector system may be considered as consisting of a symmetrical detector section which extends across a segment between angles $(-\gamma_m+\alpha,\gamma_m-\alpha)$ and an asymmetrical detector section which extends across a segment between angles $(\gamma_m-\alpha,\gamma_m)$. The asymmetrical detector section enlarges the field of view relative to the symmetrical section by an amount $\alpha$, and only a comparatively small number of detector elements need be added, i.e. at only one side of the symmetrical detector section.

Using the asymmetrical detector system, density profiles are acquired two or more times around the object, the longitudinal position in which said density profiles are acquired changing as the direction wherefrom the density profiles are measured changes. By rotating the detector system two or more times around the patient, it is easy to acquire density profiles with a range of at least $4\pi$ radians; because all individual detector cells of the detector system are rotated twice around the patient, it is very simple to register from which measured data values the computed data values are derived, because the computed data values are derived from data values measured by the same detector cell but during different revolutions around the patient. Density profiles having a range of at least $4\pi$ radians can also be acquired by rotating the detector system around the patient so far that data values from at least $4\pi$ radians are measured by a detector cell. Thus, less time will be required to acquire the density profiles, because the detector system need be rotated less far around the patient; however, in that case it is somewhat more complex to register from which individual detector cells the measured data values must be used so as to derive the computed data values. From the density profiles acquired by means of the asymmetrical detector system there is derived an image which represents a cross-section of the patient along a plane which is referred to as the scanning plane. The scanning plane does not extend parallel to the longitudinal direction and is preferably oriented approximately perpendicularly to the longitudinal direction. By performing such a volume scan it is achieved that density profiles are measured from directions all around the object to both sides of the scanning plane in a given longitudinal position. This enables computed density profiles to be derived from measured density profiles in longitudinal positions which do not excessively deviate from the longitudinal position of the cross-section along the scanning plane whose image is being formed. The appearance of streak-like disturbances in the image derived from the computed density profiles is thus avoided. The computer tomography device can thus form an image of high diagnostic quality of the patient to be examined. Small details of low contrast are clearly visible in such a high quality image; specifically, such details are not obscured by streak-like disturbances which do not pertain to image information of the relevant cross-section of the patient.

These and other advantages of a computer tomography device according to the invention are achieved by means of the embodiments defined in the dependent Claims.

Computed data values are preferably derived from preferably all data values measured from the same direction by means of the asymmetrical detector section. Each of the measured data values represents a degree of absorption of X-rays in the object to be examined from respective directions. Because the longitudinal position in which the data values are measured changes when the direction changes, for example because the patient to be examined is displaced in the longitudinal direction, the data values measured are functions of the direction and the longitudinal position. The data values measured by the asymmetrical detector section, to both sides of the scanning plane and from substantially the same directions, relate to longitudinal positions which hardly differ. The respective longitudinal positions notably do not differ by more than the displacement of the patient as it occurs during one revolution of the X-ray source with the detector system. The symmetrical detector section measures data values for which the longitudinal positions to both sides of the scanning plane differ even less. Therefore, the computation of data values of the computed density profiles utilizes only data values from longitudinal positions which do not differ significantly. Notably streak-like distortions are thus avoided in the image. The more data values measured from substantially the same direction are used to form the computed data values, the more measured data values in substantially the same longitudinal positions to both sides of the scanning plane will be used to form the computed density profiles. Therefore, preferably all data values measured by the asymmetrical detector section from the same directions to both sides of the scanning plane are used to form the computed density profiles. Preferably, the effect of data values on the computed data values is greater as they have been measured nearer to the scanning plane. It is thus achieved that the cross-section whereto the image actually relates corresponds to a thin slice, so that small details which extend a small distance in the longitudinal direction are suitably visible in the image. If redundancy occurs within the measured data values, the noise level of the density profiles can be reduced by combining corresponding data values, for example by taking the mean value thereof so as to form the computed density profiles.

It has been found that interpolation is a simple and accurate method for forming the computed data values from the measured data values.

Measurement of density profiles to both sides of the detector plane from directions all around the object to be examined by means of the asymmetrical detector, enables computation of the computed density profiles by utilizing data values which have been measured in substantially the same direction to both sides of the scanning plane in longitudinal positions which differ only slightly. Because the same data values are measured when they are measured from opposite directions, using the symmetrical detector section it suffices to measure density profiles from directions in a range of $\pi$ radians plus the angle of aperture of the symmetrical detector section. It has been found that it is not necessary to measure from directions all around the object by means of the symmetrical detector section. Data values measured from opposite directions by means of the symmetrical detector section ideally would have the same value if the longitudinal positions in which the data values are measured were the same. These longitudinal positions differ in the case of volume scanning, but for data values measured by means of the symmetrical detector section the differences between longitudinal positions of data values measured from opposite directions to both sides of the scanning plane are only small. These differences are so small that in practice they cause hardly any or no streak-like disturbances in the image, or that such streak-like disturbances are counteracted by the interpolation of data values. Using comparatively few measurements, computed density profiles can thus be formed wherefrom an image of high diagnostic quality can be derived.

It is a further object of the invention to provide a method for a computer tomography device whereby notably the appearance of "streaks" in the image is counteracted. This object is achieved by means of a method according to the invention which is characterized in that

- the density profiles are measured by means of an asymmetrical detector array from directions in a range of at least $4\pi$ radians.

These and other aspects of the invention will be described in detail hereinafter on the basis of the following embodiments and with reference to the accompanying drawing; therein:

Fig. 1 is a three-dimensional diagrammatical view of a computer tomography device according to the invention;
Fig. 2 is a diagrammatic front evaluation from the longitudinal direction of a computer tomography device according to the invention;
Fig. 3 shows a Radon diagram illustrating how computed data values are formed from measured data values for different projection angles and detector angles.

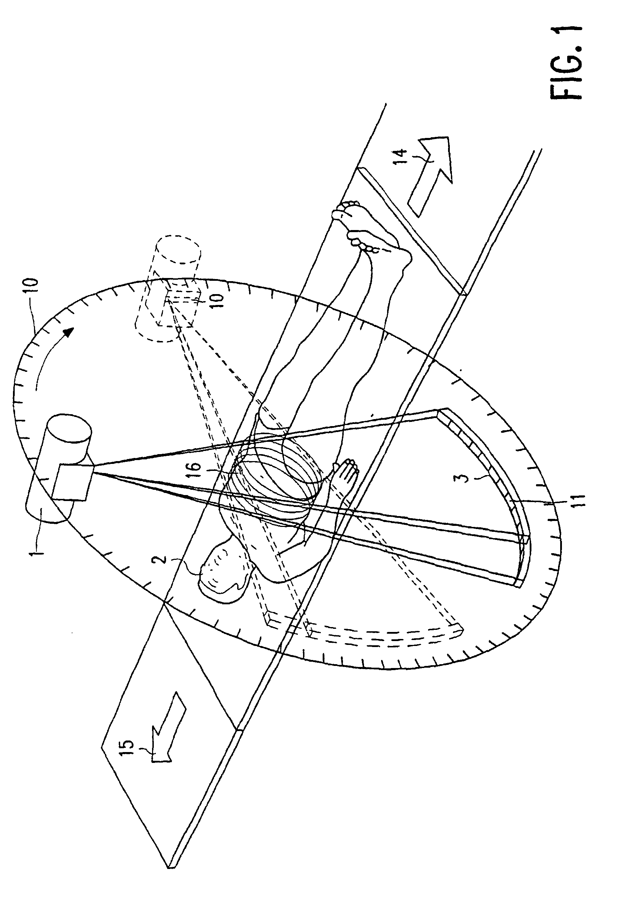

Fig. 1 is a three-dimensional diagrammatic view of a computer tomography device according to the invention. In co-operation with a slit-shaped diaphragm 10, an X-ray source 1 emits a diverging, flat (fan-shaped) X-ray beam so as to irradiate the object 2, for example a patient to be examined. The detector system 3 is arranged so as to face the X-ray source 1. The detector system of the present embodiment is a position-sensitive detector system which includes an array of individual detector cells 11. The detector cells 11 are, for example gas-filled (Xenon) detectors or solid-state detectors. The thickness of the fan-shaped X-ray beam generally is between 1 mm and 10 mm, measured halfway between the X-ray source and the X-ray detector. The intensity of the radiation which has passed the patient and is incident on the detector system is determined mainly by the absorption within the patient 2 who is arranged on a table 12 between the X-ray source and the detector system. The absorption is measured along a large number of lines by rotating the X-ray source 1 and the detector system 3 together around the patient by means of a carrier frame 13. The combined rotation of the X-ray source and the detector system may be continuous but also intermittent. Furthermore, during irradiation and rotation the patient is displaced, preferably at a uniform speed, along the axis of rotation, being the longitudinal direction indicated by the arrows 14, 15, so that the detector system acquires data from a significant three-dimensional volume of the patient. The measured density profiles are used to form images of cross-sections of the patient 2. These cross-sections are taken along parallel slices 16 of the body of the patient. Furthermore, use can also be made of an X-ray source in the form of a ring-shaped anode which is arranged around the patient, the target of an electron beam whereby X-rays are generated from the anode material then moving along the ring-shaped anode around the patient.

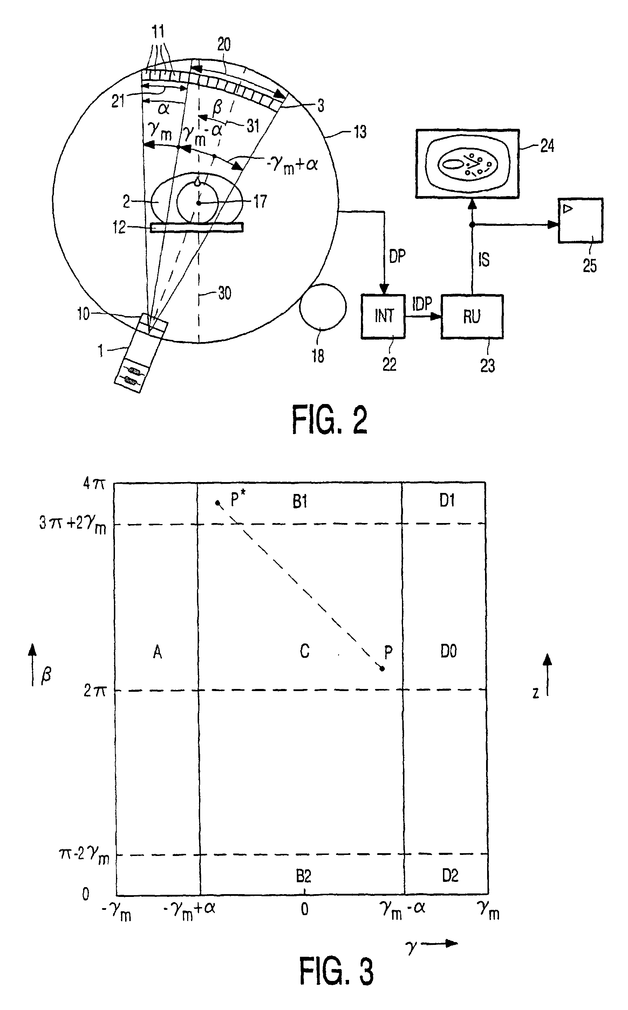

Fig. 2 is a diagrammatic front evaluation in the longitudinal direction of a computer tomography device according to the invention. The detector system 3 and the X-ray source 1 can be rotated together around a patient about a center of rotation 17. To this end, the X-ray source 1 and the detector 3 are suspended in a rotatable carrier frame 13 which is driven by means of a drive 18 which includes a motor. A reference line 30 has a fixed direction, being the vertical direction in the present example, and passes through the center of rotation 17. The projection angle $\beta$ is the angle enclosed by a central line 31 through the center of the symmetrical detector section 20 with respect to the fixed reference line 30. The position of the detector system is represented by the projection angle $\beta$. The individual detector cells 11 enclose a detector angle $\gamma$ relative to the central line 31. The symmetrical detector section 20 comprises the detector cells with a detector angle in the interval $(-\gamma_m, \gamma_m)$.

The asymmetrical detector section 21 comprises a segment of magnitude $\alpha$ with detector cells having a detector angle in the interval $(\gamma_m - \alpha, \gamma_m)$. There are no detector cells for detector angles in the interval $(-\gamma_m, -\gamma_m + \alpha)$. Because the detector cells extend across a segment which is an amount $\alpha$ larger than the symmetrical detector section, the field of view is increased in relation to the field of view of the symmetrical detector section per se, but no additional detector cells are required in the interval $(-\gamma_m, -\gamma_m + \alpha)$.

The longitudinal position z, in which the data values are measured, is dependent on the projection angle since the patient is displaced in the longitudinal direction while the X-ray source and the detector system rotate around the patient. If the rotation is uniform, i.e. rotation at a constant angular speed relative to the center of rotation 17, and if the displacement is also uniform, i.e. displacement at a constant linear speed in the longitudinal direction 15, the longitudinal position z varies linearly as a function of the projection angle $\beta$.

The detector system comprises a symmetrical detector section 20 and an asymmetrical detector section 21. The individual detector cells of the detector system measure data values which represent the X-ray absorption in successive directions through the patient. The detector system thus measures density profiles in the successive positions of the detector system. The detector system successively measures density profiles for respective values of $\beta$. The measured density profiles (DP) are applied to the data processing unit 22. The data processing unit 22 computes the computed data values by interpolation of measured data values and forms the computed data profiles on the basis thereof. The respective computed data profiles represent, each time for a projection angle $\beta$, X-ray absorption across the interval $(-\gamma_m + \alpha, \gamma_m)$ of detector angles through a slice 16 through the body of the patient in a given longitudinal position. The computed density profiles IDP are applied to the reconstruction unit 23 which forms the image of the cross-section of the patient therefrom in the longitudinal position z for which the computed density profiles (IDP) have been computed. The reconstruction unit 23 applies a mathematical Radon transformation to the computed density profiles so as to derive the brightness or grey values of the image of the cross-section of the body of the patient. The reconstruction unit notably forms an electronic image signal IS whose signal levels represent the brightness values of the image. The electronic image signal IS is applied to a monitor 24 in order to display the image thereon; the electronic image signal can also be applied to a buffer unit 24 for temporary storage of the image while awaiting further image processing or printing in the form of a hard copy.

The measured density profiles (DP) comprise a set of measured data values $P(\beta, \gamma, z)$. Because the longitudinal position z is linearly dependent on the projection angle $\beta$, the data values are measured for changing longitudinal positions; therefore, it holds for the measured data values that $P(\beta, \gamma, z) = P[\beta, \gamma \mid z(\beta)]$, where $z(\beta) = k(\beta - 2\pi) + z_0$. In order to compute data values $P_i$ for a scanning plane in the longitudinal position $z = z_0$, measured data values to both sides of the scanning plane are interpolated:

$$P_i(\beta_1, \gamma_1 \mid z_0) = w_1 P[\beta_1, \gamma_1 \mid z(\beta_1)] + w_2 P[\beta_2, \gamma_2 \mid z(\beta_2)],$$

where $\beta_2 = \beta_1 + 2\pi$ and $\gamma_1 = \gamma_2$, and the interpolated data values $P_i$ are computed for the projection angle $\beta_1$ which corresponds to the longitudinal position $z_0$ of the scanning plane, so $\beta_1 - 2\pi = [z(\beta) - z_0]/k$. Furthermore, the weights $w_1$ and $w_2$ are chosen to be such that the interpolation does not cause disturbances in the image. The weights $w_{1,2}$ are, for example, functions of the projection angle and the detector angle. Furthermore, the weights are normalized: $w_1(\beta_1, \gamma_1) + w_2(\beta_2, \gamma_2) = 1$. It has been found that the weights preferably vary linearly as a function between the difference of the projection angle of the measured data value and the projection angle at which the longitudinal position is situated in the scanning plane. It is to be noted that such an interpolation is known per se from the article "Computed tomography scanning with simultaneous patient translation" by Carl R. Crawford and Kevin F. King in Medical Physics 17 (1990) 967-982 for data values measured by means of a symmetrical detector system. If the patient were not displaced relative to the X-ray source and the detector system during rotation of the X-ray source and the detector system around the patient, if no movements were to occur inside the patient, if there were no fluctuations of the intensity of the X-rays and if there were no differences in sensitivity of the detector cells, it would not be important whether a data value is measured by measuring the absorption from a given direction or from the opposite direction. Consequently, ideally a periodicity exists for the data values measured by means of the symmetrical detector section: $P(\beta, \gamma) = P(\beta + \pi - 2\gamma, -\gamma)$ for $-\gamma_m \leq \gamma \leq \gamma_m$. In this ideal situation it suffices to vary the projection angle across a range $(0, \beta + 2\gamma_m)$. When a volume scan is performed, deviations of the periodicity occur, for example because the longitudinal position z changes upon variation of the projection angle $\beta$. It has been found that for data values measured by means of the symmetrical detector section the deviations of the periodicity are so small that they cause hardly any or no disturbances in the image. Therefore, the symmetrical detector section need only measure data values for values of the projection angle in a range $(\pi + 2\gamma_m)$ to both sides of the scanning plane. Data values, not measured but still necessary for the interpolation of data values to both sides of the scanning plane can then be accurately approximated by utilizing the periodicity. It has been found that for data values measured by means of the asymmetrical detector section it is necessary to measure data values actually to both sides of the scanning plane at projection angles in a range amounting to $2\pi$.

Fig. 3 shows a Radon diagram illustrating how computed data values are formed from measured data values at different projection angles and detector angles. Fig. 3 shows a part of the $(\beta,\gamma)$ plane with the projection angles $\beta$ and detector angles $\gamma$. In the example shown in Fig. 3, the scanning plane is taken in conformity with a projection angle $\beta_{sp} = 2\pi$. For detector angles in the interval $(-\gamma_m+\alpha,\gamma_m)$ data values are measured for values of the projection angle $\beta$ in a range around $\beta_{sp} = 2\pi$. Each of the measured data values is measured for different values of the longitudinal position $z = z_0+k(\beta-2\pi)$. No data values are measured in the range A, where $-\gamma_m \leq \gamma \leq -\gamma_m+\alpha$; this is because the range A corresponds to the range of detector angles where the asymmetrical detector system does not comprise detector cells. In the range C, in which the data values are measured by the symmetrical detector section, computed data values are obtained for $z = z_0$ by interpolation of measured data values with projection angles to both sides of $\beta_{sp} = 2\pi$, so with longitudinal positions to both sides of the scanning plane. In the regions B1 and B2, instead of measuring the data values, suitable approximations of the data values in these regions can be obtained by utilizing the almost perfect periodicity. As is indicated in Fig. 3, for a data value $P^*(\beta,\gamma)$ with $(\beta,\gamma)$ in the region B1, the measured data value $P(\beta-\pi+2\gamma_m,-\gamma)$ is taken. By refraining from the values of data values measured in the regions B1,2 it is avoided that interpolation takes place between data values which have been measured for significantly different values of the projection angle $\beta$, so of the longitudinal position z, thus avoiding the appearance of streaks. Moreover, it is achieved that the slice thickness of the cross-section reproduced by the image is comparatively small, so that details having small longitudinal dimensions are nevertheless clearly reproduced in the image. In the region C data values are measured by means of the symmetrical section of the detector system. Therefore, suitable weights for the interpolation of data values in the region C are the weights which are known per se for volume scanning by means of a symmetrical detector, as given in the article "Computed tomography scanning with simultaneous patient translation" by Carl R. Crawford and Kevin F. King in Medical Physics 17 (1990) 967-982.

In the regions D0,1,2 data values are measured by means of the asymmetrical detector section. Computed data values for detector angles in the range $(\gamma_m-\alpha,\gamma_m)$ and $z = z_0$ are interpolated between measured data values in the regions D0,1,2. It is notably not possible to derive the necessary data values in the regions D1,2 from a periodicity relation, because the corresponding values of the projection and detector angles $(\beta,\gamma)$ are situated in the region A in which the asymmetrical detector system does not comprise detector cells.

In order to prevent disturbances from occurring in the image due to discontinuities of the weights, notably at the boundaries between the regions B1 and D1, between C and D0, or between B2 and D2, it is useful to redefine the weights in the vicinity of such boundaries in such a manner that the weights are contiguous, or at least change over gradually, in such neighboring regions. It has been found that suitable results are obtained by providing such a gradual transition between weights in a vicinity of the relevant boundary whose size corresponds to approximately some tens (for example, 20) of detector cells. Such a gradual transition is made, for example by linear interpolation or a higher-order interpolation, such as cubic spline, of the weights in neighboring regions in the vicinity of the boundary.

The invention is as defined in the appended set of claims.

## Claims

1. A computer tomography device which includes

   - an X-ray source (1) for irradiating an object (2) from a plurality of directions by means of X-rays,
   - a detector system (3) for measuring density profiles of the object from a plurality of directions,
   - a reconstruction unit (23) for deriving an image signal from the density profiles,

     - the X-ray source (1), the detector system (3) and the object (2) being displaceable relative to one another in a longitudinal direction, transversely of the X-rays, during the measurement of the density profiles,

   **characterized in that**

   - the detector system (3) includes an asymmetrical system of detectors, and
   - is adapted to measure the density profiles from directions having a range of at least $4\pi$ radians.

2. A computer tomography device as claimed in Claim 1 and including

   - a data processing unit which is arranged
   - to select a scanning plane and
   - to compute computed density profiles in the scanning plane from respective measured density profiles in longitudinal positions to both sides of the scanning plane,
   - the reconstruction unit being arranged to derive the image signal from the computed density profiles,

   **characterized in that**

   - each of the measured density profiles in longitudinal positions to both sides of the scanning plane relates to directions having a range of at least $2\pi$ radians.

3. A computer tomography device as claimed in Claim 2, in which

- the detector system includes a symmetrical detector section and an asymmetrical detector section,
- the density profiles contain data values,

  - there being provided a respective data value for respective directions of X-rays and longitudinal positions, relative to the object,

- the data processing unit is arranged to compute computed data values from data values and to form the computed density profiles by means of the computed data values,

**characterized in that**

- the data processing unit is arranged to

  - compute computed data values from data values which have been measured by means of the asymmetrical detector section in respective longitudinal positions to both sides of the scanning plane and with substantially the same directions, and to form the computed density profiles by means of the computed data values.

4. A computer tomography device as claimed in Claim 3, **characterized in that**

- the data processing unit is arranged

  - to compute the computed data values from substantially all data values which have been measured by means of the asymmetrical detector section in respective longitudinal positions to both sides of the scanning plane and with substantially the same directions, and to form the computed density profiles by means of the computed data values.

5. A computer tomography device as claimed in Claim 3, in which the data processing unit is arranged to form the computed data values by interpolation of data values which have been measured in longitudinal positions to both sides of the scanning plane.

6. A computer tomography device as claimed in Claim 2, **characterized in that**

- the detector system comprises a symmetrical detector section and an asymmetrical detector section and the data processing unit is arranged to compute the computed density profiles from

- density profiles measured to both sides of the scanning plane by means of the asymmetrical detector section and from directions having a range of essentially $2\pi$ to both sides of the scanning plane, and

  - density profiles measured to both sides of the scanning plane by means of the symmetrical detector section and from directions having a range which amounts to essentially $\pi$ radians, increased by the angle of aperture of the symmetrical detector section, to both sides of the scanning plane.

7. A computer tomography method in which

- an X-ray source irradiates an object by means of X-rays from a plurality of directions,
- density profiles of the object are measured from a plurality of directions,
- an image signal is derived from the density profiles, and
- the object and the X-ray source are displaced relative to one another, transversely of the direction of the X-rays, during the measurement of the density profiles,

**characterized in that**

- the density profiles are measured by means of an asymmetrical system of detectors,
- from directions having a range of at least $4\pi$ radians.

**Patentansprüche**

1. Computertomographievorrichtung, mit:

- einer Röntgenquelle (1), um ein Objekt (2) aus einer Vielzahl von Richtungen mit Hilfe von Röntgenstrahlen zu bestrahlen,
- einem Detektorsystem (3), um Dichteprofile des Objekts aus einer Vielzahl von Richtungen zu messen,
- einer Rekonstruktionseinheit (23) zum Ableiten eines Bildsignals aus den Dichteprofilen,

  - wobei die Röntgenquelle (1), das Detektorsystem (3) und das Objekt (2) während der Messung der Dichteprofile relativ zueinander in einer Längsrichtung, quer zu den Röntgenstrahlen, verlagerbar sind,

**dadurch gekennzeichnet, dass**

- das Detektorsystem (3) ein asymmetrisches System aus Detektoren enthält und
- zum Messen der Dichteprofile aus Richtungen mit einem Bereich von zumindest 4π Radiant ausgebildet ist.

2. Computertomographievorrichtung nach Anspruch 1 und mit

- einer Datenverarbeitungseinheit, die ausgebildet ist

    - eine Abtastebene zu selektieren und
    - berechnete Dichteprofile in der Abtastebene aus jeweiligen gemessenen Dichteprofilen in Längspositionen zu beiden Seiten der Abtastebene zu berechnen,
    - wobei die Rekonstruktionseinheit ausgebildet ist, das Bildsignal aus den berechneten Dichteprofilen abzuleiten,

**dadurch gekennzeichnet, dass**

- jedes der gemessenen Dichteprofile in Längspositionen zu beiden Seiten der Abtastebene sich auf Richtungen mit einem Bereich von zumindest 2π Radiant bezieht.

3. Computertomographievorrichtung nach Anspruch 2, in der

- das Detektorsystem einen symmetrischen Detektorabschnitt und einen asymmetrischen Detektorabschnitt enthält,
- die Dichteprofile Datenwerte enthalten,

    - ein jeweiliger Datenwert für jeweilige Richtungen von Röntgenstrahlen und Längspositionen, relativ zum Objekt, vorgesehen ist,

- die Datenverarbeitungseinheit ausgebildet ist, berechnete Datenwerte aus Datenwerten zu berechnen und mit Hilfe der berechneten Datenwerte die berechneten Dichteprofile zu bilden,

**dadurch gekennzeichnet, dass**

- die Datenverarbeitungseinheit ausgebildet ist,

    - berechnete Datenwerte aus Datenwerten zu berechnen, die mit Hilfe des asymmetrischen Detektorabschnitts in jeweiligen Längspositionen zu beiden Seiten der Abtastebene und bei nahezu den gleichen Richtungen gemessen worden sind, und mit Hilfe der berechneten Datenwerte die berechneten Dichteprofile zu bilden.

4. Computertomographievorrichtung nach Anspruch 3, **dadurch gekennzeichnet, dass**

- die Datenverarbeitungseinheit ausgebildet ist

    - die berechneten Datenwerte aus nahezu allen Datenwerten zu berechnen, die mit Hilfe des asymmetrischen Detektorabschnitts in jeweiligen Längspositionen zu beiden Seiten der Abtastebene und bei nahezu den gleichen Richtungen gemessen worden sind, und mit Hilfe der berechneten Datenwerte die berechneten Dichteprofile zu bilden.

5. Computertomographievorrichtung nach Anspruch 3, in der die Datenverarbeitungseinheit ausgebildet ist, um die berechneten Datenwerte durch Interpolation von Datenwerten zu bilden, die in Längspositionen zu beiden Seiten der Abtastebene gemessen worden sind.

6. Computertomographievorrichtung nach Anspruch 2, **dadurch gekennzeichnet, dass**

- das Detektorsystem einen symmetrischen Detektorabschnitt und einen asymmetrischen Detektorabschnitt umfasst und die Datenverarbeitungseinheit ausgebildet ist zum Berechnen der berechneten Dichteprofile aus

    - zu beiden Seiten der Abtastebene mit Hilfe des asymmetrischen Detektorabschnitts und aus Richtungen mit einem Bereich von im Wesentlichen 2π zu beiden Seiten der Abtastebene gemessenen Dichteprofilen und
    - zu beiden Seiten der Abtastebene mit Hilfe des symmetrischen Detektorabschnitts und zu beiden Seiten der Abtastebene aus Richtungen mit einem Bereich, der im Wesentlichen π Radiant beträgt, erhöht um den Öffnungswinkel des symmetrischen Detektorabschnitts, gemessenen Dichteprofilen.

7. Computertomographieverfahren, in dem

- eine Röntgenquelle ein Objekt mit Hilfe von Röntgenstrahlen aus einer Vielzahl von Richtungen bestrahlt,

- Dichteprofile des Objekts aus einer Vielzahl von Richtungen gemessen werden,
- ein Bildsignal aus den Dichteprofilen abgeleitet wird und
- das Objekt und die Röntgenquelle während der Messung der Dichteprofile relativ zueinander, quer zur Richtung der Röntgenstrahlen, verlagert werden

**dadurch gekennzeichnet, dass**

- die Dichteprofile mit Hilfe eines asymmetrischen Systems aus Detektoren gemessen werden,
- aus Richtungen mit einem Bereich von zumindest 4π Radiant.

**Revendications**

1. Dispositif de tomographie à ordinateur qui comprend

   - une source de rayons X (1) pour l'irradiation d'un objet (2) à partir d'une pluralité de directions à l'aide de rayons X,
   - un système de détecteurs (3) servant à mesurer des profils de densité de l'objet à partir d'une pluralité de directions,
   - une unité de reconstruction (23) servant à dériver un signal d'image à partir des profils de densité.

     - la source de rayons X (1), le système de détecteurs (3) et l'objet (2) pouvant être déplacés les uns par rapport aux autres dans une direction longitudinale, de façon transversale par rapport aux rayons X, pendant la mesure des profils de densité,

   **caractérisé en ce que**

   - le système de détecteurs (3) comprend un système asymétrique de détecteurs, et est conçu pour mesurer les profils de densité à partir de directions présentant une gamme d'au moins 4 π radians.

2. Dispositif de tomographie à ordinateur selon la revendication 1, et comprenant une unité de traitement de données qui est conçu

   - pour sélectionner un plan de balayage et
   - pour calculer des profils de densité calculés dans le plan de balayage à partir de profils de densité mesurés respectifs dans des positions longitudinales des deux côtés du plan de balayage,

- l'unité de reconstruction état conçue pour dériver le signal d'image à partir des profils de densité calculés,

**caractérisé en ce que**

- chacun des profils de densité mesurés dans les positions longitudinales des deux côtés du plan de balayage concerne des directions présentant une gamme d'au moins 2 π radians.

3. Dispositif de tomographie à ordinateur selon la revendication 2, dans lequel

   - le système de détecteurs comprend une section de détection symétrique et une section de détection asymétrique,
   - les profils de densité contiennent des valeurs de données,

     - une valeur de données respective étant prévue pour les directions respectives de rayons X et les positions longitudinales, par rapport à l'objet,

   - l'unité de traitement de données est conçue pour calculer des valeurs de donnés calculées à partir de valeurs de données et pour former les profils de densité calculés à l'aide des valeurs de données calculées,

   **caractérisé en ce que**

   - l'unité de traitement de données est conçue pour

     - calculer des valeurs de données calculées à partir des valeurs de données qui sont mesurées à l'aide de la section de détection asymétrique dans des positions longitudinales respectives des deux côtés du plan de balayage et avec pratiquement les mêmes directions, et pour former les profils de densité calculés à l'aide des valeurs de données calculées.

4. Dispositif de tomographie à ordinateur selon la revendication 3, **caractérisé en ce que**

   - l'unité de traitement de données est conçue

     - pour calculer les valeurs de données calculées à partir de pratiquement toutes les valeurs de données qui sont mesurées à l'aide de la section de détection asymétrique dans des positions longitudinales respectives des deux côtés du plan de balayage et avec pratiquement les mêmes directions, et pour former les profils de densité calculés à l'aide des valeurs de données calculées.

5. Dispositif de tomographie à ordinateur selon la revendication 3, dans laquelle l'unité de traitement de données est conçue pour former les valeurs de données calculées par interpolation de valeurs de données qui sont mesurées dans des positions longitudinales des deux côtés du plan de balayage.

6. Dispositif de tomographie à ordinateur selon la revendication 2, **caractérisé en ce que**

   - le système de détecteurs comprend une section de détection symétrique et une section de détection asymétrique et l'unité de traitement de données est conçue pour calculer les profils de densité calculés à partir

      - des profils de densité mesurés des deux côtés du plan de balayage à l'aide de la section de détection asymétrique et à partir de directions présentant une gamme qui est essentiellement de 2 π des deux côtés du plan de balayage, et
      - des profils de densité mesurés des deux côtés du plan de balayage à l'aide de la section de détection symétrique et à partir de directions présentant une gamme qui est essentiellement de π radians, augmentée de l'angle d'ouverture de la section de détection symétrique, des deux côtés du plan de balayage.

7. Procédé pour la tomographie à ordinateur selon lequel

   - une source de rayons X assure l'irradiation d'un objet à l'aide de rayons X provenant d'une pluralité de directions,
   - -des profils de densité de l'objet sont mesurés à partir d'une pluralité de directions,
   - -un signal d'image est dérivé des profils de densité, et
   - l'objet et la source de rayons X sont déplacés l'un par rapport à l'autre, de façon transversale par rapport à la direction des rayons X, pendant la mesure des profils de densité,

   **caractérisé en ce que**

   - -les profils de densité sont mesurés à l'aide d'un système de détection asymétrique,
   - à partir de directions présentant une gamme d'au moins 4 π radians.

FIG. 1

FIG. 2

FIG. 3